# EUROPEAN PATENT APPLICATION

(11) **EP 2 537 454 A2**
(43) Date of publication of application: **26.12.2012**
(21) Application number: 12004600.8
(22) Date of filing: 19.06.2012
(51) Int. Cl.: A61B 1/00, A61B 1/04

(54) **Electronic endoscope apparatus**

(30) Priority: 20.06.2011 JP 2011136408
(71) Applicant: Olympus Corporation, Shibuya-ku, Tokyo (JP)
(72) Inventor: Tanaka, Satoshi, Tokyo (JP); Azuma, Motoo, Tokyo (JP); Nishimura, Hisashi, Tokyo (JP); Kotoda, Kaoru, Tokyo (JP); Takizawa, Kazuhiro, Tokyo (JP); Sato, Takayuki, Tokyo (JP); Kobayashi, Naruyasu, Tokyo (JP)
(74) Representative: Gunzelmann, Rainer

(57) **Abstract**

The image processor (20) includes a display oscillation circuit (206) that generates a display clock, and a monitor synchronization signal generating part (207) that generates a monitor display synchronization signal based on the display clock. The endoscope (10) includes an imaging oscillation circuit (111) that generates an imaging clock, pixels (115) that are driven based on the imaging clock, converts optical information into electrical signals and outputs the electrical signals as a digital data of a serial form, and a phase comparator (113) that compare the phase of the monitor display synchronization signal with the imaging clock, and control the oscillation of the imaging oscillation circuit (111).

## Description

### BACKGROUND OF THE INVENTION

### Field of the Invention

The present invention relates to an electronic endoscope apparatus.

### Description of Related Art

In recent years, the number of pixels of solid-state imaging devices, such as a CCD (Charge Coupled Device) and a CMOS (Complementary Metal Oxide Semiconductor) sensor, has increased with the advance of semiconductor technology. This tendency is not an exception even in electronic endoscope apparatuses, and the number of pixels of solid-state imaging devices included in the electronic endoscope apparatuses is increasing. However, with an increase in the number of pixels of the solid-state imaging devices, the frequency of a clock signal that is required for image processing has also become higher, and causing various problems as a result. For example, in the structure of an electronic endoscope apparatus, the distal end portion of a scope on which a solid-state imaging device is mounted, and an image processor that performs image processing are separated, and signal degradation on a transmission path between the solid-state imaging device and the image processor has a tendency to occur. Additionally, if the frequency of the clock signal becomes high, the influence of signal degradation on the transmission path between the solid-state imaging device and the image processor becomes greater still. Additionally, leakage of electromagnetic waves caused by high frequency signals flowing through the transmission path between the solid-state imaging device and the image processor also becomes more significant.

As a method of solving such a problem, an electronic endoscope apparatus described in Japanese Unexamined Patent Application, First Publication No. 2001-275956 is suggested. FIG 5 is a block diagram showing the configuration of an electronic endoscope apparatus that is known in the related art. In the illustrated example, a high frequency noise emitted between an electronic scope 900 and a processor device 950 is suppressed by inserting a waveform smoothing circuit 916 at an output stage of the electronic scope 900.

However, the viewpoint of the synchronization between the electronic scope (endoscope) and the monitor (image processor) is not described in Japanese Unexamined Patent Application, First Publication No. 2001-275956. Since imaging devices with various field angles are mounted on the endoscope in accordance with targets to be observed or applications, every endoscope has a different operating frequency or field angle. Hence, in order to display a moving image captured by the endoscope on the monitor, a frequency conversion matched with the synchronization signal of the monitor is required. Additionally, the endoscope captures a moving image at a timing based on an imaging clock, and the monitor displays the moving image at a timing based on a display clock.

However, when the frequency conversion is performed, a cycle by which the electronic scope captures a one-frame image is subtly different from a cycle by which the monitor displays a one-frame image according to the relationship between the imaging clock and the display clock. Therefore, a problem occurs in that the phases of both deviate gradually. If the deviation between the phases accumulates and the deviation between both phases exceeds a cycle of one frame, this leads to phenomena, such as "passing" and "dropping".

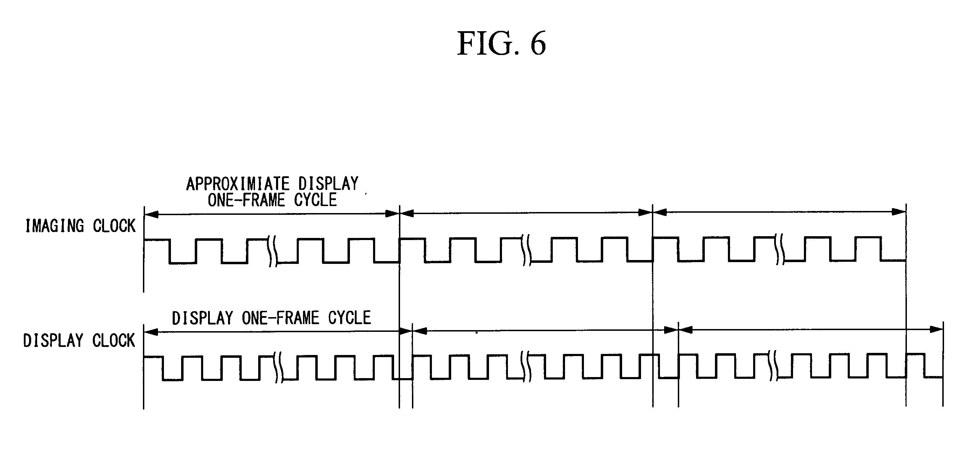

FIG. 6 is a schematic view showing the relationship between the cycle of one frame based on the imaging clock and the cycle of one frame based on the display clock. As illustrated, the phases of the imaging clock and the display clock are different. Therefore the cycle of one frame based on the imaging clock and the cycle of one frame based on the display clock deviate slightly from each other. Although there is a slight deviation in one frame, as illustrated, the deviation accumulates as time passes. If the deviation exceeds a cycle of one frame, this leads to phenomena, such as "passing" and "dropping".

On the other hand, since an increase in speed accompanying an increase in definition has also progressed on the monitor side, there is a need to satisfy strict timing regulations in the input of signals to the monitor. Even if one-frame cycles can be completely matched between imaging and display, when a synchronization signal is generated based on a clock on the endoscope side that is not based on television standards, normal display may not be achieved in the monitor.

### SUMMARY OF THE INVENTION

The invention has been made in consideration of the above circumstances, and an object thereof is to provide an electronic endoscope apparatus that can secure the synchronization between imaging and display.

According to a first aspect of the present invention, an electronic endoscope apparatus includes an image processor having a display clock generating part that generates a display clock, and a monitor synchronization signal generating part that generates a monitor display synchronization signal based on the display clock; an endoscope having an imaging clock generating part that generates an imaging clock, a solid-state imaging device that is driven based on the imaging clock and converts and outputs optical information into electrical signals, and a phase comparison oscillation control part that compares the phase of the monitor display synchronization signal and the imaging clock, and controls the oscillation of the imaging clock generating part.

Additionally, according to an electronic endoscope apparatus of a second aspect of the present invention, in the above first aspect, the image processor may include a clock recovery part that receives a digital data of a serial form that the solid-state imaging device outputs, and reproduces a transmission clock from the digital data.

Additionally, according to an electronic endoscope apparatus of a third aspect of the present invention, in the above first aspect, the solid-state imaging device may be of a CMOS type, and the imaging clock generating part, the phase comparison oscillation control part, and the solid-state imaging device may be mounted in the same semiconductor chip.

Additionally, according to an electronic endoscope apparatus of a fourth aspect of the present invention, in the above first aspect, the endoscope may include a differential signal generating part, the image processor may include a differential signal receiving part, and the endoscope and the image processor may transmit and receive the digital data using differential signals.

Additionally, according to an electronic endoscope apparatus of a fifth aspect of the present invention, in the above first aspect, the endoscope may include an electrooptic conversion part, the image processor may include a photoelectric conversion part, and the endoscope and the image processor may transmit and receive the digital data using optical signals.

Additionally, according to an electronic endoscope apparatus of a sixth aspect of the present invention, in the above first aspect, the endoscope may include a radio transmitting part, the image processor may include a radio receiving part, and the endoscope and the image processor may transmit and receive the digital data by radio communication.

Additionally, according to an electronic endoscope apparatus of a seventh aspect of the present invention, in the above first aspect, the endoscope may include a compression part that compresses the digital data, and the image processor may include an expansion part that expands the digital data compressed by the compression part.

According to the above, the display clock generating part generates a display clock. Additionally, the monitor synchronization signal generating part generates a monitor display synchronization signal based on the display clock. Additionally, the solid state imaging device converts and outputs optical information into electrical signals. Additionally, the imaging clock generating part generates an imaging clock that becomes the basis upon which the solid-state imaging device is driven. Additionally, the phase comparison oscillation control part compares the phase of the monitor display synchronization signal with the phase of the imaging clock, and controls the oscillation of the imaging clock generating part. Thereby, since the monitor display synchronization signal and the imaging clock can be synchronized, the synchronization between imaging and display can be secured.

### BRIEF DESCRIPTION OF THE DRAWINGS

FIG. 1 is a block diagram showing the configuration of an electronic endoscope apparatus in a first embodiment of the present invention.
FIG. 2A is a schematic view showing the relationship between imaging clocks and monitor display synchronization signals before a phase comparator controls the frequency of the imaging clocks in the first embodiment of the present invention.
FIG 2B is a schematic view showing the relationship between imaging clocks and monitor display synchronization signals after the phase comparator controls the frequency of the imaging clocks in the first embodiment of the present invention.
FIG. 3 is a block diagram showing the configuration of an electronic endoscope apparatus in a second embodiment of the present invention.
FIG 4 is a block diagram showing the configuration of an electronic endoscope apparatus in a third embodiment of the present invention.

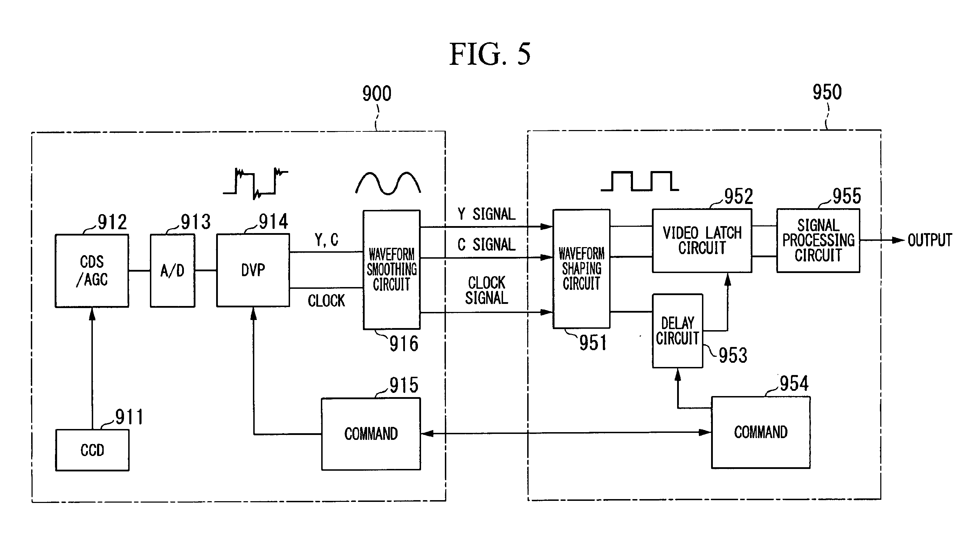

FIG. 5 is a block diagram showing the configuration of an electronic endoscope apparatus that is known in the related art.
FIG. 6 is a schematic view showing the relationship between the cycle of one frame based on an imaging clock and the cycle of one frame based on a display clock.

### DETAILED DESCRIPTION OF THE INVENTION

### (First Embodiment)

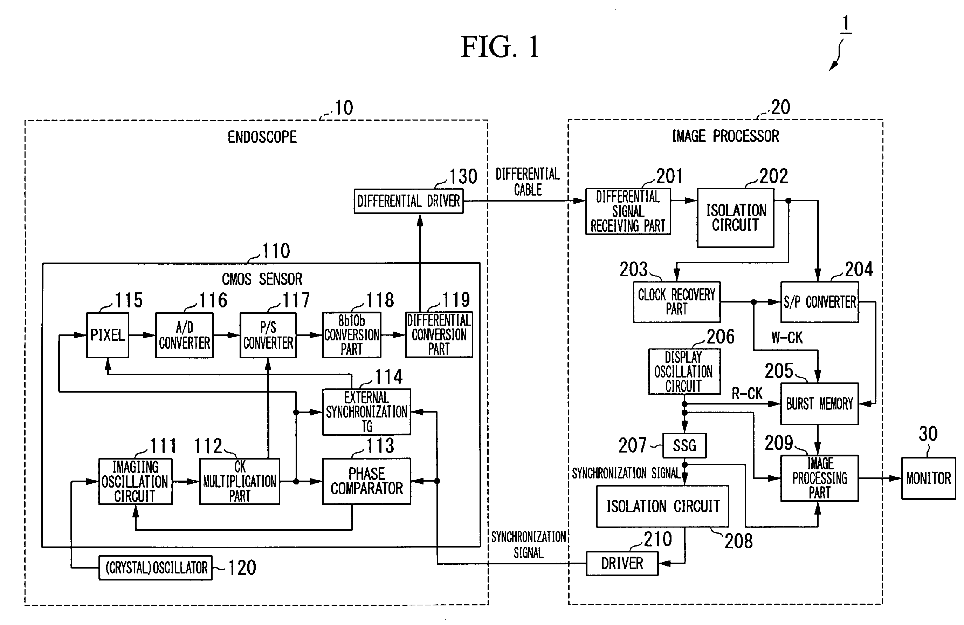

A first embodiment of the present invention will be described below with reference to the accompanying drawings. FIG. 1 is a block diagram showing the configuration of an electronic endoscope apparatus in the present embodiment. In the illustrated example, the electronic endoscope apparatus 1 includes an endoscope 10, an image processor 20, a monitor 30, and a light source device that is not shown. The monitor 30, which is a liquid crystal display or the like, displays an image (moving image). The light source device generates light with which a subject is irradiated.

The endoscope 10 includes a CMOS sensor 110, an oscillator 120, and a differential driver 130. The CMOS sensor 110 includes an imaging oscillation circuit 111 (imaging clock generating part), a CK multiplication part 112 (clock multiplication part), a phase comparator 113 (phase comparison oscillation control part), an external synchronization TG 114 (external synchronization timing generator), pixels 115, an A/D converter 116 (analog digital converter), a P/S converter 117 (parallel serial converter), and an 8b10b conversion part 118. Additionally, the endoscope 10 may include a differential conversion part 119 (differential signal generating part).
Additionally, a solid-state imaging device is of, for example, a CMOS type, corresponds to, for example, the pixels 115, the A/D converter 116, and the P/S converter 117, and converts and outputs an optical information into electrical signals. Additionally, the imaging oscillation circuit 111, the phase comparator 113, and the solid-state imaging device may be mounted in the same semiconductor chip.

The oscillator 120 is, for example, a crystal oscillator, and oscillates at a natural frequency. The imaging oscillation circuit 111 generates an imaging clock on the basis of the oscillation of the oscillator 120. The CK multiplication part 112 multiplies the imaging clock generated by the imaging oscillation circuit 111. The phase comparator 113 receives a monitor display synchronization signal from the image processor 20. Additionally, the phase comparator 113 compares the phase of the imaging clock with the phase of the monitor display synchronization signal. Here, the phase of the imaging clock is the phase of a clock multiplied by the CK multiplication part 112 after being generated by the imaging oscillation circuit 111, and the phase of the monitor display synchronization signal is the phase of a signal output from the image processor 20. The phase comparator 113 controls the oscillation of the imaging oscillation circuit 111 so that the phase of the imaging clock and the phase of the monitor display synchronization signal coincide. That is, the phase comparator 113 controls the frequency of the imaging clocks that the imaging oscillation circuit 111 outputs. The external synchronization TG 114 receives the monitor display synchronization signal from the image processor 20. Additionally, the external synchronization TG 114 generates an imaging synchronization signal that instructs the imaging timing of the pixels 115 on the basis of the received monitor display synchronization signal so that the pixels 115 capture a one-frame image in the same cycle as a cycle by which the image processor 20 displays a one-frame image on the monitor 30.

The pixels 115 are driven on the basis of the imaging clock, that is, are operated by the imaging clock that is generated by the imaging oscillation circuit 111 and multiplied by the CK multiplication part 112. The pixels 115 also output image data (capture a one-frame image) according to incident light, at a timing based on the imaging synchronization signal generated by the external synchronization TG 114. The image data that the pixels 115 output is analog signals. The A/D converter 116 converts the analog signals that the pixels 115 output into digital signals, and converts the analog signals into parallel signals having a predetermined number of bits. The P/S converter 117 converts the parallel signals converted by the A/D converter 116 into serial signals. The 8b10b conversion part 118 increases the number of bits of the serial signals converted by the P/S converter, and encodes the serial signals converted by the P/S converter 117 so that the same signal level among the serial signals does not continue for a predetermined period or more. Thereby, even if only image data is transmitted from the endoscope 10 to the image processor 20, it is easy to recover and generate a clock on the image processor 20 side. The differential conversion part 119 converts the serial signals encoded by the 8b10b conversion part 118 into differential signals. The differential driver 130 transmits the differential signals converted by the differential conversion part 119 to the image processor 20 via a differential cable. That is, the endoscope 10 and the image processor 20 transmit and receive digital data using the differential signals.

The image processor 20 includes isolation circuits 202 and 208, a clock recovery part 203, an S/P converter 204 (serial parallel converter), a burst memory 205, a display oscillation circuit 206 (display clock generating part), a monitor synchronization signal generating part 207 (SSG), an image processing part 209, and a driver 210. The image processor 20 may further include a differential signal receiving part 201. Additionally, the image processor 20 may include a frame memory instead of the burst memory 205.

The differential signal receiving part 201 receives the differential signals transmitted via the differential cable from the endoscope 10. The isolation circuits 202 and 208 maintain a dielectric strength voltage between the image processor 20 and the endoscope 10. The clock recovery part 203 generates a write clock (W-CK) from a signal transmitted from the differential signal receiving part 201. That is, the clock recovery part 203 may receive a digital data of a serial form that the solid-state imaging device outputs, and may reproduce a transmission clock from this digital data. The S/P converter 204 converts serial signals converted by the differential signal receiving part 201 into parallel signals. The burst memory 205 stores the parallel signals converted by the S/P converter 204, that is, image data, on the basis of the write clock generated by the clock recovery part 203.

The display oscillation circuit 206 is, for example, a crystal oscillator (XO), and oscillates at natural frequency to generate a display clock. The monitor synchronization signal generating part 207 generates monitor display synchronization signals (a vertical synchronization signal and a horizontal synchronization signal) on the basis of the display clock generated by the display oscillation circuit 206. The image processing part 209 reads image data from the burst memory 206 on the basis of a read clock (R-CK) on the basis of the oscillation of the display oscillation circuit 206. Moreover, the image processing part 209 causes an image (one-frame image) based on the image data to be displayed on the monitor 30, at a timing based on the monitor display synchronization signal generated by the monitor synchronization signal generating part 207. The driver 210 transmits the monitor display synchronization signal generated by the monitor synchronization signal generating part 207 to the endoscope 10.

Next, a method of synchronizing a cycle by which the endoscope 10 captures a one-frame image with a cycle by which the image processor 20 causes the one-frame image to be displayed on the monitor 30 will be described.

The display oscillation circuit 206 of the image processor 20 generates a display clock. The monitor synchronization signal generating part 207 generates a monitor display synchronization signal on the basis of the display clock generated by the display oscillation circuit 206. The driver 210 transmits the monitor display synchronization signal generated by the monitor synchronization signal generating part 207 to the endoscope 10.

On the other hand, the oscillator 120 of the endoscope 10 oscillates at a natural frequency. The imaging oscillation circuit 111 generates an imaging clock on the basis of the oscillation of the oscillator 120. The CK multiplication part 112 multiplies the imaging clock generated by the imaging oscillation circuit 111. The phase comparator 113 compares the phase of the imaging clock with the phase of the monitor display synchronization signal. Here, the phase of the imaging clock is the phase of a clock multiplied by the CK multiplication part 112 after being generated by the imaging oscillation circuit 111. The phase of the monitor display synchronization signal is the phase of a signal transmitted from the image processor 20. The phase comparator 113 controls the oscillation of the imaging clock that the imaging oscillation circuit 111 outputs so that the phase of the imaging clock and the phase of the monitor display synchronization signal coincide. That is, the phase comparator controls the frequency of the imaging clocks that the imaging oscillation circuit 111 outputs. In other words, the phase comparator 113 controls the frequency of the imaging clocks that the imaging oscillation circuit 111 outputs so that the leading edge of the imaging clock multiplied by the CK multiplication part 112 after being generated by the imaging oscillation circuit 111, and the leading edge of the monitor display synchronization signal are in the same phase. More specifically, the frequency of the imaging clock is controlled so that the leading edge of thecycle (for example, the vertical synchronization signal and the horizontal synchronization signal), which is generated from each of a clock signal that generates the monitor display synchronization signal and the imaging clock coincide.
Thereby, the phase of the imaging clock multiplied by the CK multiplication part 112 after being generated by the imaging oscillation circuit 111, and the phase of the monitor display synchronization signal transmitted from the image processor 20 coincide. Here, the imaging control signal (for example, the vertical synchronization signal and the horizontal synchronization signal) is generated so that the vertical synchronization signal generated from the monitor display synchronization signal and the horizontal synchronization signal generated from the imaging clock coincide. Accordingly, a frame rate of the monitor display and a frame rate of the image data output become the same.

Here, although a configuration in which the monitor display synchronization signal is transmitted to the endoscope as it is has been described, the monitor display synchronization signal may not be a signal generated at exactly the same timing as a synchronization signal to be input to an actual monitor, and only has to be a timing signal generated such that the frame rate of the monitor display and the frame rate of the captured image data output become the same. For example, a configuration may be adopted in which only a portion in which the cycles of horizontal synchronization signals become the same is output as synchronization signals so that frame cycles become the same.

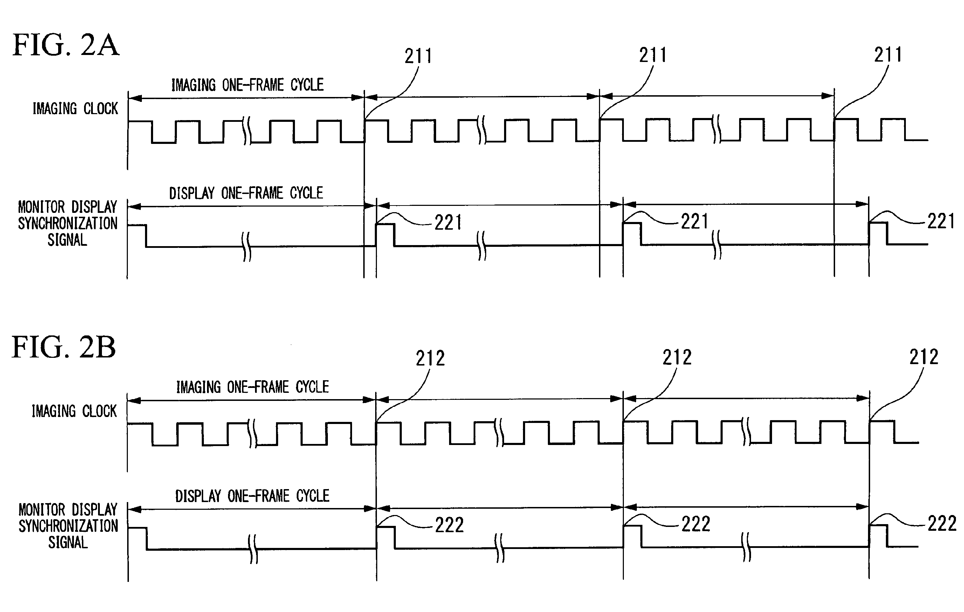

FIGs. 2A and 2B are schematic views showing the relationship between imaging clocks and monitor display synchronization signals before and after the phase comparator 113 controls the frequency of the imaging clocks in the present embodiment. FIG 2A is a schematic view showing the relationship between imaging clocks and monitor display synchronization signals before the phase comparator 113 controls the frequency of the imaging clocks. In the illustrated example, the timing of leading edges 211 of the imaging clocks and the timing of leading edges 221 of the monitor display synchronization signals deviate from each other. Therefore, a cycle by which the image processor 20 causes one image to be displayed on the monitor 30, and a cycle by which the endoscope 10 captures one image cannot be completely matched (an imaging cycle and a display cycle cannot be completely matched).

FIG 2B is a schematic view showing the relationship between imaging clocks and monitor display synchronization signals after the phase comparator 113 controls the frequency of the imaging clocks. In the illustrated example, the timing of leading edges 212 of the imaging clocks and the timing of leading edges 222 of the monitor display synchronization signals coincide. Therefore, the cycle by which the image processor 20 causes one image to be displayed on the monitor 30, and the cycle by which the endoscope 10 captures one image can be completely matched (the imaging cycle and the display cycle can be completely matched).

The pixels 115 of the endoscope 10 are operated using the imaging clock multiplied by the CK multiplication part 112 after being generated by the imaging oscillation circuit 111, and capture a one-frame image at a timing based on the imaging synchronization signal generated by the external synchronization TG 114. Additionally, the image processing part 209 of the image processor 20 causes the one-frame image to be displayed on the monitor 30, at a timing based on the monitor display synchronization signal generated by the monitor synchronization signal generating part 207. At this time, since the phase of the imaging clock and the phase of the monitor display synchronization signal coincide, the cycle by which the endoscope 10 captures a one image and the cycle by which the image processor 20 causes the one image to be displayed on the monitor 30 can be completely matched. Here, the phase of the imaging clock is the phase of a clock multiplied by the CK multiplication part 112 after being generated by the imaging oscillation circuit 111. Additionally the phase of the monitor display synchronization signal is the phase of a signal generated by the monitor synchronization signal generating part 207.

Accordingly, the electronic endoscope apparatus 1 can secure the cycle by which the endoscope 10 captures an image, and the cycle by which the image processor 20 causes the image to be displayed on the monitor 30, even when the processing speed of the CMOS sensor 110 is set to be high. Thereby, the electronic endoscope apparatus 1 can prevent phenomena, such as "passing" and "dropping". Here, in order to simplify description, the timing control of performing general image processing, such as correction processing, color conversion processing, and filter processing of the data (RAW) of the imaging device is omitted.

### (Second Embodiment)

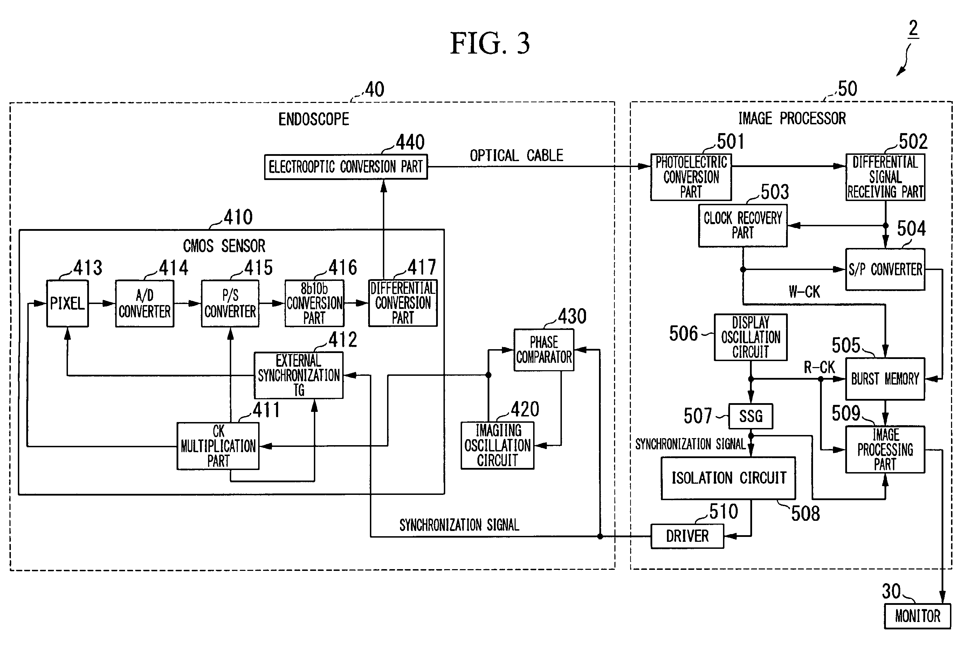

Next, a second embodiment of the present invention will be described. FIG. 3 is a block diagram showing the configuration of an electronic endoscope apparatus in the present embodiment. In the illustrated example, the electronic endoscope apparatus 2 includes an endoscope 40, an image processor 50, a monitor 30, and a light source device that is not shown. The monitor 30 is the same as that of the monitor 30 in the first embodiment.

The endoscope 40 includes a CMOS sensor 410, an imaging oscillation circuit 420, and a phase comparator 430. The CMOS sensor 410 includes a CK multiplication part 411, an external synchronization TG 412, pixels 413, an A/D converter 414, a P/S converter 415, an 8b10b conversion part 416, and a differential conversion part 417. Additionally, the endoscope 40 may further include an electrooptic conversion part 440.

The imaging oscillation circuit 420 generates an imaging clock for driving the CMOS sensor 410. The phase comparator 430 compares the phase of the imaging clock generated by the imaging oscillation circuit 420 with the phase of a monitor display synchronization signal transmitted from the image processor 50. The phase comparator 430 controls the oscillation of the imaging oscillation circuit 420 so that the phase of the imaging clock generated by the imaging oscillation circuit 420 and the phase of the monitor display synchronization signal coincide. That is, the phase comparator 430 controls the frequency of the imaging clocks that the imaging oscillation circuit 420 outputs. The CK multiplication part 411 multiplies the imaging clock generated by the imaging oscillation circuit 420.

The external synchronization TG 412, the pixels 413, the A/D converter 414, the P/S converter 415, the 8b10b conversion part 416, and the differential conversion part 417 are the same as those of the respective parts in the first embodiment. The electrooptic conversion part 440 converts the differential signals converted by the differential conversion part 417 into optical signals and transmits the converted optical signals to the image processor 50 via an optical cable. That is, the endoscope 40 and the image processor 50 transmit and receive digital data using the optical signals.

The image processor 50 includes a differential signal receiving part 502, a clock recovery part 503, an S/P converter 504, a burst memory 505, a display oscillation circuit 506, a monitor synchronization signal generating part 507, an isolation circuit 508, an image processing part 509, and a driver 510. Additionally, the image processor 50 may further include a photoelectric conversion part 501. Additionally, the image processor 50 may include a frame memory instead of the burst memory 505.

The photoelectric conversion part 501 receives the optical signals transmitted from the endoscope 40 via the optical cable, and converts the received optical signals into differential signals. The differential signal receiving part 502 converts the differential signals converted by the photoelectric conversion part 501 into serial signals. The clock recovery part 503, the S/P converter 504, the burst memory 505, the display oscillation circuit 506, the monitor synchronization signal generating part 507, the isolation circuit 508, the image processing part 509, and the driver 510 are the same as those of the respective parts in the first embodiment. Here, although an example in which the output signals from the photoelectric conversion part are differential signals is shown, the differential signal receiving part 502 may be an electrical signal receiving part that receives the signals output from the photoelectric conversion part.

Additionally, although not shown, the endoscope 40 includes a movable insertion part to be inserted into a body cavity, a manipulating part continuously provided at a proximal end portion of the insertion part, and a universal cord connected to the image processor 50 or the like. A proximal end of the universal cord is coupled to a scope connector. The scope connector is of a composite type. The image processor 50 and the light source device are connected to the scope connector. A distal end portion in which the CMOS imaging device 410 for imaging the inside of a body cavity, or the like is built is continuously provided at the distal end of the insertion part. The manipulating part or the scope connector includes the imaging oscillation circuit 420 and the phase comparator 430.

Additionally, the electronic endoscope apparatus 2 of the present embodiment is different from the electronic endoscope apparatus 1 in the first embodiment in that the CMOS sensor 410 does not have the imaging oscillation circuit 420 built therein. Additionally, the imaging oscillation circuit 420 is constituted by, for example, a crystal oscillation module that can vary a frequency referred to as VCXO (Voltage Controlled Xtal Oscillator). Additionally, the electrooptic conversion part 440 is connected to the CMOS sensor 410, and has a form in which data is transmitted via the optical cable from the endoscope 40 to the image processor 50.

In addition, an example in which the imaging oscillation circuit 420 and the phase comparator 430 are arranged in the manipulating part or the scope connector in which there are comparatively few restrictions to the size of members to be arranged, has been shown in the above-described example. However, the present invention is not limited to this, and the imaging oscillation circuit 420 and the phase comparator 430 may be arranged in the vicinity of the CMOS sensor 410, that is, in the distal end portion. Additionally, a configuration may be adopted in which the configuration shown in the present embodiment and the configuration shown in the first embodiment are combined. For example, a form in which data is transmitted via the optical cable from the endoscope 40 to the image processor 50 is shown in the present embodiment. However, a form in which data is transmitted via the differential cable may be adopted. Additionally, a configuration may be adopted in which a synchronization signal to be output from the image processor 50 to the endoscope 40 is transmitted using a differential signal in order to reduce influence of jitter or the like.

Next, a method of synchronizing a cycle by which the endoscope 40 captures a one-frame image with a cycle by which the image processor 50 causes the one-frame image to be displayed on the monitor 30 will be described.

The display oscillation circuit 506 of the image processor 50 generates a display clock. The monitor synchronization signal generating part 507 generates a monitor display synchronization signal on the basis of the display clock generated by the display oscillation circuit 506. The driver 510 transmits the monitor display synchronization signal generated by the monitor synchronization signal generating part 507 to the endoscope 40.

On the other hand, the imaging oscillation circuit 420 of the endoscope 40 generates an imaging clock. The phase comparator 430 compares the phase of the imaging clock generated by the imaging oscillation circuit 420 with the phase of the monitor display synchronization signal input from the image processor 50, and controls the oscillation of the imaging oscillation circuit 420 so that the phase of the imaging clock generated by the imaging oscillation circuit 420 and the phase of the monitor display synchronization signal coincide with. That is, the phase comparator 430 controls the frequency of the imaging clocks output by the imaging oscillation circuit 420. In other words, the phase comparator 430 controls the frequency of the imaging clocks output by the imaging oscillation circuit 420 so that the leading edge of the imaging clock generated by the imaging oscillation circuit 420, and the leading edge of the monitor display synchronization signal are in the same phase. Thereby, the phase of the imaging clock generated by the imaging oscillation circuit 420 and the phase of the monitor display synchronization signal input from the image processor 50 coincide.

The CK multiplication part 411 of the endoscope 40 multiplies the imaging clock that is generated by the imaging oscillation circuit 420 and that has a phase synchronized with the monitor display synchronization signal. In addition, since the phase of the imaging clock before multiplication by the CK multiplication part 411 and the phase of the monitor display synchronization signal coincide, the imaging clock after the CK multiplication part 411 multiplies also coincides with the phase of the monitor display synchronization signal.

The pixels 413 are operated using the imaging clock multiplied by the CK multiplication part 411 after being generated by the imaging oscillation circuit 420, and capture a one-frame image at a timing based on the imaging synchronization signal generated by the external synchronization TG 412. Additionally, the image processing part 509 of the image processor 50 causes the one-frame image to be displayed on the monitor 30, at a timing based on the monitor display synchronization signal generated by the monitor synchronization signal generating part 507. At this time, since the phase of the imaging clock multiplied by CK multiplication part 411 after being generated by the imaging oscillation circuit 420 and the phase of the monitor display synchronization signal generated by the monitor synchronization signal generating part 507 coincide, the cycle by which the endoscope 40 captures a one image and the cycle by which the image processor 50 causes the one image to be displayed on the monitor 30 can be completely matched.

Accordingly, the electronic endoscope apparatus 2 can secure the cycle by which the endoscope 40 captures an image, and the cycle by which the image processor 50 causes the image to be displayed on the monitor 30, even when the processing speed of the CMOS sensor 410 is set to be high. Thereby, the electronic endoscope apparatus 2 can prevent phenomena, such as "passing" and "dropping".

Additionally, since the electronic endoscope apparatus 2 converts electrical signals output by the CMOS sensor 410 into optical signals and transmits the optical signals via the optical cable when image data is transmitted from the endoscope 40 to the image processor 50, the apparatus is not easily influenced by noise disturbance. Therefore, the capacity of image data of the electronic endoscope apparatus 2 increases as the performance of the pixels 413 becomes high. As a result, even if the transmission speed of image data from the endoscope 40 to the image processor 50 is set to be high, the image data can be transmitted in a state where the image data is not easily influenced by noise disturbance. Additionally, since the electronic endoscope apparatus 2 can be operated so that the CMOS sensor 410 is synchronized with a monitor display synchronization signal to be used for a display, the phase from imaging to display can be fixed. Moreover, monitor display with little display delay is made possible by making the image processing part 509 and processing timing cooperate. Additionally, since a path along which image data is transmitted from the endoscope 40 to the image processor 50 is not an electrical signal line, isolation circuits can be reduced.

### (Third Embodiment)

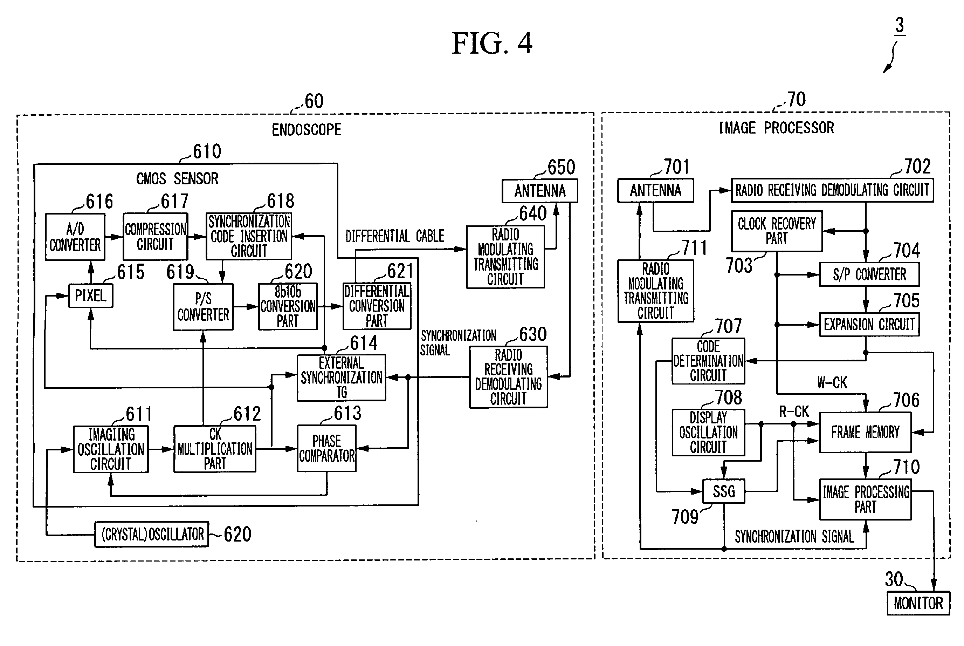

Next, a third embodiment of the present invention will be described. FIG. 4 is a block diagram showing the configuration of an electronic endoscope apparatus in the present embodiment. In the illustrated example, the electronic endoscope apparatus 3 includes an endoscope 60, an image processor 70, a monitor 30, and a light source device that is not shown. The monitor 30 is the same as that of the monitor 30 in the first embodiment.

The endoscope 60 includes a CMOS sensor 610, an oscillator 620, the radio receiving demodulating circuit 630, a radio modulating transmitting circuit 640, and an antenna 650. Additionally, a radio transmitting part related to a claim corresponds to, for example, the radio modulating transmitting circuit 640 and the antenna 650. The CMOS sensor 610 includes an imaging oscillation circuit 611, a CK multiplication part 612, a phase comparator 613, an external synchronization TG 614, pixels 615, an A/D converter 616, a synchronization code insertion circuit 618, a P/S converter 619, a 8b10b conversion part 620, and a differential conversion part 621. Additionally, the endoscope 60 may further include a compression circuit 617 (compression part).

The oscillator 620, the imaging oscillation circuit 611, the CK multiplication part 612, the phase comparator 613, the external synchronization TG 614, the pixels 615, the A/D converter 616, the 8b10b conversion part 620, and the differential conversion part 621 are the same as those of the respective parts in the first embodiment. The compression circuit 617 compresses parallel signals converted by the A/D converter 616. The synchronization code insertion circuit 618 inserts an imaging synchronization code based on an imaging synchronization signal generated by the external synchronization TG 614 into the parallel signals compressed by the compression circuit 617. The P/S converter 619 converts the parallel signals into which the synchronization code insertion circuit 618 has inserted the imaging synchronization code into serial signals. The radio modulating transmitting circuit 640 modulates differential signals converted by the differential conversion part 621, and generates radio modulation signals.

The antenna 650 transmits and receives radio signals with an external device. Specifically, the antenna 650 receives radio modulation signals transmitted from the image processor 70. Additionally, the antenna 650 transmits the radio modulation signals converted by the radio modulating transmitting circuit 640 to the image processor 70. That is, the endoscope 60 and the image processor 70 transmit and receive digital data by radio communication. The radio receiving demodulating circuit 630 demodulates the radio modulation signals received by the antenna 650, and acquires a monitor display synchronization signal.

The image processor 70 includes an antenna 701, a radio receiving demodulating circuit 702, a clock recovery part 703, an S/P converter 704, a frame memory 706, a code determining circuit 707, a display oscillation circuit 708, a monitor synchronization signal generating part 709, an image processing part 710, and a radio modulating transmitting circuit 711. Additionally, a radio receiving part related to a claim corresponds to, for example, the antenna 701 and the radio receiving demodulating circuit 702. The display oscillation circuit 708 and the monitor synchronization signal generating part 709 are the same as those of the respective parts in the first embodiment. Additionally, the image processor 70 may include an expansion circuit 705 (expansion part).

The antenna 701 transmits and receives radio signals with an external device. Specifically, the antenna 701 receives radio modulation signals transmitted from the endoscope 60. Additionally, the antenna 701 transmits the radio modulation signals modulated by the radio modulating transmitting circuit 711 to the endoscope 60. The radio receiving demodulating circuit 702 demodulates the radio modulation signals received by the antenna 701, and acquires serial signals. The clock recovery part 703 generates a clock from the serial signals acquired by the radio receiving demodulating circuit 702. The S/P converter 704 converts the serial signals acquired by the radio receiving demodulating circuit 702 into parallel signals. The expansion circuit 705 expands the digital data compressed by the compression circuit 617, and expands the parallel signals converted by the S/P converter 704. The frame memory 706 stores the parallel signals expanded by the expansion circuit 705, that is, image data, on the basis of a write clock (W-CK) generated by the clock recovery part 703. The code determining circuit 707 determines the head of the image data on the basis of the parallel signals expanded by the expansion circuit 705.

The image processing part 710 reads image data from the frame memory 706 on the basis of a read clock (R-CK) on the basis of the oscillation of the display oscillation circuit 708. Additionally, the image processing part 710 causes an image (one-frame image) based on the image data to be displayed on the monitor 30, at a timing based on the monitor display synchronization signal generated by the monitor synchronization signal generating part 709. The radio modulating transmitting circuit 711 modulates the monitor display synchronization signal generated by the monitor synchronization signal generating part 709, and generates radio modulation signals.

Additionally, although not shown, the endoscope 60 includes a movable insertion part to be inserted into a body cavity, and a manipulating part continuously provided at a proximal end portion of the insertion part. A distal end portion in which the CMOS sensor 610 for imaging the inside of a body cavity, or the like is built is continuously provided at the distal end of the insertion part.

Although an example in which the CMOS sensor 610 has the imaging oscillation circuit 611 built therein is shown in the above-described example, the present invention is not limited to this, and as shown in the second embodiment, a configuration may be adopted in which a crystal oscillation module (VCXO) that can vary frequency is arranged outside the CMOS sensor 610 (except the distal end portion). Additionally, a configuration may be adopted in which the configuration shown in the present embodiment and the configurations shown in the first and second embodiments are combined.

A method of synchronizing a cycle by which the endoscope 60 captures one-frame image and a cycle by which the image processor 70 causes the one-frame image to be displayed on the monitor 30 is the same as the synchronizing method in the first embodiment. Accordingly, the electronic endoscope apparatus 3, similar to the electronic endoscope apparatus 1 in the first embodiment, can secure the cycle by which the endoscope 60 captures an image, and the cycle by which the image processor 70 causes the image to be displayed on the monitor 30, even when the processing speed of the CMOS sensor 610 is set to be high. Thereby, the electronic endoscope apparatus 3 can prevent phenomena, such as "passing" and "dropping".

Additionally, since the electronic endoscope apparatus 3 transmits image data using radial communication when the image data is transmitted from the endoscope 60 to the image processor 70, the apparatus is not easily influenced by noise disturbance. Therefore, the capacity of image data of the electronic endoscope apparatus 3 increases as the performance of the pixels becomes high. As a result, even if the transmission speed of image data from the endoscope 60 to the image processor 70 is set to be high, the image data can be transmitted in a state where the image data is not easily influenced by noise disturbance. Additionally, since a path along which image data is transmitted from the endoscope 60 to the image processor 70 is wireless, the diameter of the endoscope 60 can be reduced and isolation circuits can be reduced.

Although the first to third embodiments of the present invention have been described hitherto in detail with reference to the drawings, specific configurations are not limited to the embodiments, and the present invention also includes various designs without departing from the scope of the present invention.

## Claims

1. An electronic endoscope apparatus comprising:
an image processor having
a display clock generating part that generates a display clock, and
a monitor synchronization signal generating part that generates a monitor display synchronization signal based on the display clock;
an endoscope having
an imaging clock generating part that generates an imaging clock,
a solid-state imaging device that is driven based on the imaging clock and converts and outputs an optical information into electrical signals; and
a phase comparison oscillation control part that compares the phase of the monitor display synchronization signal and the imaging clock, and controls the oscillation of the imaging clock generating part.

2. The electronic endoscope apparatus according to Claim 1,
wherein the image processor includes a clock recovery part that receives a digital data of a serial form output by the solid-state imaging device, and reproduces a transmission clock from the digital data.

3. The electronic endoscope apparatus according to Claim 1, wherein
the solid-state imaging device is of a CMOS type; and
the imaging clock generating part, the phase comparison oscillation control part, and the solid-state imaging device are mounted in the same semiconductor chip.

4. The electronic endoscope apparatus according to Claim 1, wherein
the endoscope includes a differential signal generating part;
the image processor includes a differential signal receiving part; and
the endoscope and the image processor transmit and receive the digital data using differential signals.

5. The electronic endoscope apparatus according to Claim 1, wherein
the endoscope includes an electrooptic conversion part;
the image processor includes a photoelectric conversion part; and
the endoscope and the image processor transmit and receive the digital data using optical signals.

6. The electronic endoscope apparatus according to Claim 1, wherein
the endoscope includes a radio transmitting part;
the image processor includes a radio receiving part; and
the endoscope and the image processor transmit and receive the digital data by radio communication.

7. The electronic endoscope apparatus according to Claim 1, wherein
the endoscope includes a compression part that compresses the digital data; and
the image processor includes an expansion part that expands the digital data compressed by the compression part.
